# EUROPÄISCHE PATENTSCHRIFT

(11) **EP 0 538 783 B1**
(45) Veröffentlichungstag und Bekanntmachung des Hinweises auf die Patenterteilung: **27.09.1995**
(21) Anmeldenummer: 92117906.5
(22) Anmeldetag: 20.10.1992
(51) Int. Cl.: A61K 31/275, C07C 255/21, C07C 255/23

(54) **N-phenyl-2-cyano-3-hydroxycrotonsäureamidderivate und deren Verwendung als Arzneimittel mit immunmodulatorischer Eigenschaft**
N-phenyl-2-cyano-3-hydroxycrotonic acid amid derivatives and their use as medicaments having immunomodulating properties
Dérivés d'amides de l'acide n-phényl-cyano-2-hydroxy-3-crotonique et leur utilisation comme médicaments ayant des propriétés immunomodulants

(30) Priorität: 23.10.1991 DE 4134934
(43) Veröffentlichungstag der Anmeldung: 28.04.1993
(73) Patentinhaber: HOECHST AKTIENGESELLSCHAFT, 65926 Frankfurt am Main (DE)
(72) Erfinder: Schleyerbach, Rudolf, Dr., W-6238 Hofheim (Ts) (DE); Bartlett, Robert Ryder, Dr., W-6100 Darmstadt 12 (DE); Kuo, Elizabeth Anne, Covingham, Swindon, Wiltshire SN3 5PH (GB); Little, Edward James, Swindon, Wiltshire SN1 5QR (GB)

(56) Entgegenhaltungen:
- EP-A- 0 217 206
- EP-A- 0 257 882
- EP-A- 0 413 329
- US-A- 4 061 767

## Beschreibung

In der US-Patentschrift 4 061 767 werden Hydroxyethylidencyanessigsäureamide beschrieben, die antiphlogistische und analgetische Wirkung besitzen.

Die Verwendung von N-(4-Trifluormethylphenyl)-2-cyan-3-hydroxy-crotonsäureamid als Arzneimittel zur Behandlung von chronischen Graft-versus-Host-Krankheiten und systemischen Lupus erythematodes ist bekannt (EP-A-0 217 206).

Es besteht ein Bedürfnis an Medikamenten mit entzündungshemmender Wirkung sowie der günstigen Beeinflussung immunpathologischer Prozesse, die sich aufgrund eines günstigeren Wirkungsprofils von den bekannten Medikamenten vorteilhaft durch bessere Verträglichkeit und günstigere Verweilzeit im Körper einerseits und einen mehr kausalen Eingriff in die Entzündungsprozesse andererseits unterscheiden. Erfolgversprechende Ansatzpunkte hierfür bieten solche Pharmaka, die verstärkt die exzessive Bildung der proinflammatorischen Leukotriene unterbinden, hochreaktive Sauerstoffradikale, die als Entzündungsmediatoren die Zell- und Gewebszerstörung in den entzündlich rheumatischen Gelenken perpetuell unterhalten, desaktivieren und/oder das gestörte Immunsystem restaurieren.

Überraschenderweise zeigen N-Phenyl-2-cyano-3-hydroxycrotonsäureamidderivate der Formel I 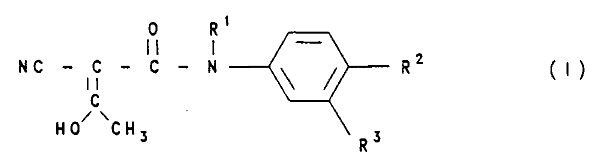
pharmakologische Eigenschaften, die die zuvor aufgestellten Anforderungen erfüllen und sich demnach hervorragend zur Behandlung von beispielsweise rheumatischer Erkrankungen, Autoimmunerkrankungen oder von Abstoßungsreaktionen des Organempfängers gegen das verpflanzte Organ eignen.

Die erfindungsgemäßen Verbindungen greifen vorteilhaft in das gestörte Immunsystem ein, wie sich durch Unterdrückung der Arthus-Reaktion und durch Normalisierung der supprimierten Immunaktivität in den pathologischen Modellen der mit Freundschem Adjuvans induzierten Arthritis demonstrieren läßt. Ferner zeigen sie eine kürzere Verweilzeit im Metabolismus des behandelten Organismus.

Die Erfindung betrifft daher die Verwendung von mindestens einem N-Phenyl-2-cyano-3-hydroxycrotonsäureamid der Formel I 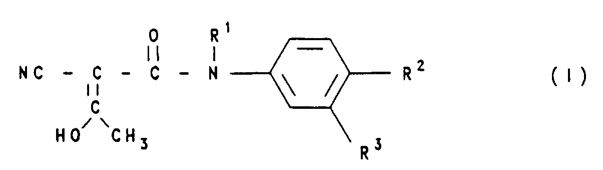
und/oder mindestens einem seiner physiologisch verträglichen Salze, wobei
R¹ ist:
a) Wasserstoffatom oder
b) (C₁-C₄)-Alkyl, geradkettig oder verzweigt,
R² ist ein Rest aus der Gruppe:
a) W(CH₂)ₙCX₃,
   W ist:
   1) Sauerstoffatom oder
   2) Schwefelatom,
   X ist Fluor, Chlor oder Jod,
   n ist eine ganze Zahl von 1 bis 3,
b) WCX₃,
c) CX₃,
d) (CH₂)ₙCX₃,
e) Fluor, Chlor oder Jod,
f) CN oder
g) NO₂,
R³ ist ein Rest aus der Gruppe:
a) (C₁-C₄)-Alkyl, geradkettig oder verzweigt,
b) (C₃-C₆)-Cycloalkyl,
c) CN oder
d) 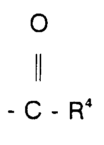 R⁴ ist:
   1) Wasserstoffatom,
   2) (C₁-C₄)-Alkyl, geradkettig oder verzweigt, oder
   3) (C₃-C₆)-Cycloalkyl,
zur Herstellung von Arzneimitteln zur Behandlung von rheumatischen Erkrankungen, Autoimmunerkrankungen oder von Abstoßungsreaktionen des Organempfängers gegen das verpflanzte Organ.

Bevorzugt wird mindestens ein N-Phenyl-2-cyano-3-hydroxycrotonsäureamid der Formel I und/oder mindestens eines seiner physiologisch verträglichen Salze verwendet, wobei
R¹ ist Wasserstoff,
R² ist ein Rest aus der Gruppe:
a) CN,
b) Halogen, wie Fluor, Chlor oder Jod,
c) CX₃,
   X ist Fluor, Chlor oder Jod, oder
d) NO₂.
R³ ist ein Rest aus der Gruppe:
a) - CH₃,
b) - CN oder
c)

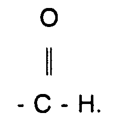

Insbesondere werden die folgenden Verbindungen der Formel I und/oder ihre physiologisch verträglichen Salze verwendet:
N-(3-Methyl-4-nitrophenyl)-2-cyano-3-hydroxycrotonsäureamid,
N-(3-Methyl-4-cyanophenyl)-2-cyano-3-hydroxycrotonsäureamid,
N-(3-Methyl-4-fluorphenyl)-2-cyano-3-hydroxycrotonsäureamid,
N-(3-Methyl-4-chlorphenyl)-2-cyano-3-hydroxycrotonsäureamid,
N-(3-Methyl-4-jodphenyl)-2-cyano-3-hydroxycrotonsäureamid oder
N-(3-Methyl-4-trifluormethylphenyl)-2-cyano-3-hydroxycrotonsäureamid.

Geeignete physiologisch verträgliche Salze der N-Phenyl-2-cyano-3-hydroxycrotonsäureamide der Formel I sind beispielsweise Alkali, Erdalkali- oder Ammoniumsalze, einschließlich solcher von physiologisch verträglichen organischen Ammoniumbasen.

Die Erfindung betrifft ferner neue N-Phenyl-2-cyano-3-hydroxycrotonsäureamide der Formel I 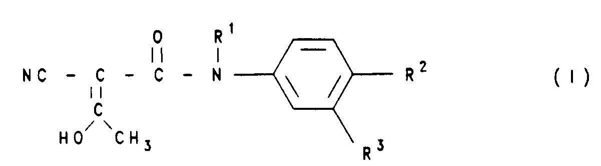
und/oder mindestens einem seiner physiologisch verträglichen Salze, wobei
R¹ ist:
a) Wasserstoffatom oder
b) (C₁-C₄)-Alkyl, geradkettig oder verzweigt,
R² ist ein Rest aus der Gruppe:
a) W(CH₂)ₙCX₃,
   W ist:
   1) Sauerstoffatom oder
   2) Schwefelatom,
   X ist Fluor, Chlor oder Jod,
   n ist eine ganze Zahl von 1 bis 3,
b) WCX₃,
c) CX₃,
d) (CH₂)ₙCX₃,
e) Fluor, Chlor oder Jod,
f) CN oder
g) NO₂,
R³ ist (C₃-C₆)-Cycloalkyl oder 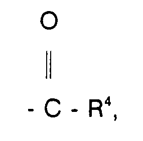
R⁴ ist:
1) Wasserstoffatom
2) (C₁-C₄)-Alkyl, geradkettig oder verzweigt, oder
3) (C₃-C₆)-Cycloalkyl.

Bevorzugt sind N-Phenyl-2-cyano-3-hydroxycrotonsäureamide der Formel I und/oder mindestens eines seiner physiologisch verträglichen Salze, wobei
R¹ ist Wasserstoff,
R² ist ein Rest aus der Gruppe:
a) CN,
b) Fluor, Chlor oder Jod,
c) CX₃,
   X ist Fluor, Chlor oder Jod, oder
d) NO₂,
R³ ist
a) Cyclopropyl oder
b) 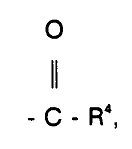
R⁴ ist Cyclopropyl.

Die N-Phenyl-2-cyano-3-hydroxycrotonsäureamidderivate der Formel I können beispielsweise nach dem folgenden Verfahren hergestellt werden:
Eine Verbindung der Formel II, 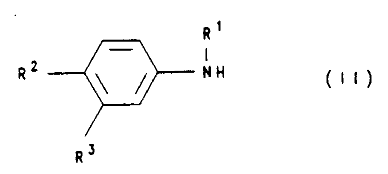
wird mit einer Verbindung der Formel III, 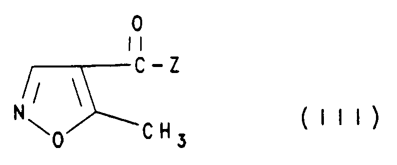
in der Z für ein Halogenatom, vorzugsweise Chlor oder Brom steht und R¹, R² und R³ die in Formel I angegebene Bedeutung haben, umgesetzt und die erhaltene Verbindung in Gegenwart eines basischen Mittels in die entsprechende Verbindung der Formel I umgesetzt.

Die obengenannten Umsetzungen zur Herstellung der Verbindungen der Formel I erfolgen unter Standardbedingungen auf bekannte Weise (US 4 061 767; EP-A-0 217 206).

Die erfindungsgemäßen Verbindungen der Formel I und deren entsprechende Salze eignen sich aufgrund ihrer wertvollen pharmakologischen Eigenschaften bei gleichzeitig hervorragender Verträglichkeit in besonderer Weise für die Verwendung als Wirkstoffe in Arzneimitteln zur Behandlung von entzündlich rheumatischen Erkrankungen. Sie können entweder für sich allein, beispielsweise in Form von Mikrokapseln, in Mischungen miteinander oder in Kombination mit geeigneten Hilfs- und/oder Trägerstoffen verabreicht werden.

Die vorliegende Erfindung betrifft ferner die Verwendung von mindestens einem N-Phenyl-2-cyano-3-hydroxycrotonsäureamid der Formel I und/oder mindestens einem seiner physiologisch verträglichen Salze zur Herstellung von Arzneimitteln zur Behandlung von Autoimmunerkrankungen, beispielsweise systemischen Lupus erythematodes oder von chronischen Graft-versus-Host-Krankheiten oder Abstoßungsreaktionen des Organempfängers gegen das verpflanzte Organ.

Unter dem Begriff Organ, werden alle Organe bei Säugetieren, insbesondere des Menschen verstanden, beispielsweise Niere, Herz, Haut, Leber, Bauchspeicheldrüse, Muskel, Knochen, Darm oder Magen, aber auch Blut oder Haare. Unter Abstoßungsreaktion sind alle Abwehrmaßnahmen des Empfängerorganismusses gemeint, die letztlich zum Zell- oder Gewebsuntergang des übertragenen Organs führen bzw. die Lebensfähigkeit des übertragenen Organs beeinträchtigen. Die Applikation erfolgt vor, während und nach einer Organverpflanzung beim Empfänger und/oder Spender.

Gegenstand der Erfindung sind somit auch Arzneimittel, die aus mindestens einem N-Phenyl-2-cyano-3-hydroxycrotonsäureamid der Formel I und/oder mindestens einem von deren entsprechenden Säureadditionssalzen bestehen oder mindestens einen dieser Wirkstoffe neben pharmazeutisch geeigneten und physiologisch verträglichen Trägerstoffen, Verdünnungsmitteln und/oder Hilfsstoffen enthalten.

Die erfindungsgemäßen Arzneimittel können oral, topisch, rektal oder gegebenenfalls auch parenteral appliziert werden, wobei die orale Anwendung bevorzugt ist.

Geeignete feste oder flüssige galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung üblicherweise Hilfsmittel, wie Trägerstoffe, Spreng-, Binde-, Überzugs-, Quellungs-, Gleit- oder Schmiermittel, Geschmacksstoffe, Süßungsmittel oder Lösungsvermittler, Verwendung finden. Als häufig verwendete Hilfsstoffe seien z.B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, Polyethylenglykole und Lösungsmittel, wie etwa steriles Wasser und ein- oder mehrwertige Alkohole, z.B. Glycerin, genannt.

Vorzugsweise werden die pharmazeutischen Präparate in Dosierungseinheiten hergestellt und verabreicht, wobei jede Einheit als aktiven Bestandteil eine bestimmte Dosis mindestens einer Verbindung gemäß Formel I und/oder mindestens eines entsprechenden Säureadditionssalzes enthält. Bei festen Dosierungseinheiten, wie Tabletten, Kapseln, Dragees oder Suppositorien, kann diese Dosis bis zu etwa 300 mg, bevorzugt jedoch etwa 100 bis 200 mg betragen.

Für die Behandlung eines an entzündlich rheumatischen Erkrankungen leidenden, erwachsenen Patienten sind - je nach Wirksamkeit der Verbindungen gemäß Formel I und/oder der entsprechenden Salze am Menschen - Tagesdosen von etwa 5 bis 300 mg Wirkstoff, vorzugsweise etwa 25 bis 100 mg, bei oraler Verabreichung indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Schließlich können die Verbindungen der Formel I und die entsprechenden Säureadditionssalze bei der Herstellung der vorgenannten galenischen Zubereitungsformen auch zusammen mit anderen geeigneten Wirkstoffen, beispielsweise Antiuricopathika, Thrombocytenaggregationshemmern, Analgetika und anderen steroidalen oder nichtsteroidalen Antiphlogistika, formuliert werden.

Die Struktur der nachstehend beschriebenen Verbindungen wurde durch Elementaranalyse, IR, sowie ¹H-NMR-Spektren gesichert.

### Beispiel 1 N-(3-Methyl-4-nitrophenyl)-2-cyano-3-hydroxycrotonsäureamid

Cyanessigsäure (1,5 g; 17,6 mmol) und Phosphorpentachlorid (4,1 g; 19,4 mmol) werden unter Rückfluß in Dichlormethan (40 ml) für 1 Stunde erhitzt. 3-Methyl-4-nitroanilin (1,6 g; 10,6 mmol) wird zugefügt und eine weitere Stunde unter Rückflußbedingungen erhitzt. Nach dem Abkühlen wird der Feststoff filtriert, mit Wasser eine weitere Stunde gerührt, gefiltert und das Filtrat unter vermindertem Druck zur Trockne gebracht. Man erhält so 2-Cyano-N(3-methyl-4-nitrophenyl)-acetamid (1,4 g; 64 %).

2-Cyano-N-(3-methyl-4-nitrophenyl)-acetamid (0,61 g; 2,8 mmol) werden in Tetrahydrofuran (30 ml) gelöst und Natriumhydrid (80 %ige Dispersion in Öl; 0,21 g; 7 mmol) wird zugefügt. Es wird bei 25°C für 15 Minuten gerührt und tropfenweise über 5 Minuten 1-Acetylimidazol (0,46 g; 4,2 mmol) zugefügt und weitere 15 Minuten gerührt. 2 ml Eisessig werden zugefügt und bei 25°C wird für 1 Stunde gerührt und anschließend in eisgekühltes Wasser (100 ml) geschüttet. Dann wird der pH der Lösung mit konzentrierter Salzsäure auf pH 1 eingestellt, gefiltert, mit Wasser gewaschen und unter vermindertem Druck zur Trockne gebracht. Man erhält so N-(3-Methyl-4-nitrophenyl)-2-cyano-3-hydroxycrotonsäureamid.
Ausbeute: 0,65 g (88 % der Theorie)
Schmelzpunkt: 231 bis 233°C
C₁₂H₁₁N₃O₄ (Molekulargewicht (MG) = 261,24)
Analog dem vorstehenden Beispiel werden die folgenden Verbindungen der Formel I hergestellt.

### Beispiel 2

### N-(3-Methyl-4-cyanophenyl)-2-cyano-3-hydroxycrotonsäureamid

Schmelzpunkt: 239 bis 240°C
C₁₃H₁₁N₃O₂ (MG = 241,25)

### Beispiel 3

### N-(3-Methyl-4-fluorphenyl)-2-cyano-3-hydroxycrotonsäureamid

Schmelzpunkt: 167,5 bis 168°C
C₁₂H₁₁FN₂O₂ (MG = 234,23)

### Beispiel 4

### N-(3-Methyl-4-chlorphenyl)-2-cyano-3-hydroxycrotonsäureamid

Schmelzpunkt: 184°C
C₁₂H₁₁ClN₂O₂ (MG = 250,69)

### Beispiel 5

### N-(3-Methyl-4-jodphenyl)-2-cyano-3-hydroxycrotonsäureamid

Schmelzpunkt: 177 bis 178°C
C₁₂H₁₁JN₂O₂ (MG = 342,14)

### Beispiel 6

### N-(3-Methyl-4-trifluormethylphenyl)-2-cyano-3-hydroxycrotonsäureamid

Schmelzpunkt: 182 bis 184°C
C₁₃H₁₁F₃N₂O₂ (MG = 284,23)

### Pharmakologische Prüfung und Ergebnisse

Die Prüfung der erfindungsgemäßen Verbindungen der Formel I auf entzündungshemmende Wirkung, sowie die Beeinflussung immunpathologischer Prozesse erfolgt in den anschließend beschriebenen Tiermodellen.
Tiermodell 1.
   Carrageenin-Pfotenödem
   Die Untersuchungen werden nach der Methode von Winter et al. (J. Pharmacol. exp. Ther. 141, 369 (1963) durchgeführt.
   Als Versuchstiere dienen ca. 17 Stunden nüchterne männliche Ratten eines CFHB-Stammes mit einem Körpergewicht zwischen 160 und 180 g. Durch subplantare Injektion (linke Pfote) von 0,2 ml 0,5 %iger Carrageenin-Suspension in 0,9 % NaCl pro Tier entsteht ein Ödem in der Umgebung der Injektionsstelle, dessen Ausmaß plethysmographisch bestimmt wird. 1 Stunde vor Carrageenin-Gabe werden die Prüfsubstanzen in Carboxymethylcellulose (CMC-Suspensionen) oral per Schlundsonde in einem Injektionsvolumen von 1 ml/100 g Körpergewicht verabreicht, sowie 3 Stunden nach dieser Gabe das Pfotenvolumen bestimmt. Zur Standardisierung der Eintauchtiefe dient eine Farbmarkierung am Tarsocruralgelenk. Anzahl der Versuchstiere: n = 6 - 12 pro dosi.
Tiermodell 2.
   Verzögerte Überempfindlichkeit beim Maus-Pfotenödem
   Mäuse (8 bis 10, männliche CD-1, Gewicht 25 bis 30 g) werden durch eine subkutane Injektion von 1 mg Methylrinderserumalbumin (MBSA) in 0,2 ml einer physiologischen kompletten Freund's Adjuvans (FCA) Emulsion sensibilisiert. Eine Kontrollgruppe erhält Injektionen von physiologischen FCA. 7 Tage nach der Sensibilisierung wird 0,1 mg MBSA in 0,05 ml einer physiologischen Kochsalzlösung in die rechte hintere Pfote injiziert. Die Pfotenödeme werden 24 Stunden später bestimmt. Als Kontrolle dienen Injektionen von physiologischer Kochsalzlösung in die linke Pfote. Die erfindungsgemäßen Verbindungen werden oral verabreicht. Verabreichungen erfolgen am 4., 5., 6. und zweimal am 7. Tag, eine Stunde vor und 6 Stunden nach der weiteren MBSA-Injektion.
Tiermodell 3.
   Verzögerte Überempfindlichkeit beim Ratten-Pfotenödem
   Ratten (8 bis 12, männliche CFHB, Gewicht 160 bis 180 g) werden durch Injektion in den Schwanz wie in Test 2 sensibilisiert. Im Unterschied zum Test 2 werden 0,4 mg Mycobacterium tuberculosis Antigen in 0,2 ml physiologischer Kochsalzlösung am 7. Tag verabreicht, sonst sind die Versuchsbedingungen wie in Test 2.

Als Beurteilungskriterium für die obengenannten Tiermodelle dient das Pfotenvolumen im Vergleich zur Kontrollgruppe. Die Hemmung der Pfotenvolumenzunahme wird in Prozentwerten angegeben.

Die Tabelle 1 zeigt die Ergebnisse; in Klammern ist die Dosis der erfindungsgemäßen Verbindungen in mg/kg angegeben.

**Tabelle 1**

| Beispiel Nr. | Tiermodell 1 | Tiermodell 2 | Tiermodell 3 |
|---|---|---|---|
| 1 | 1 (50) | 53 (100) | 90 (50) |
| 2 | -20 (50) | 41 (100) | 88 (50) |
| 3 | -1 (50) | 52 (100) | 70 (50) |
| 4 | 12 (50) | 78 (100) | 45 (50) |
| 5 | 7 (50) | n.b. | 28 (50) |
| 6 | 37 (50) | n.b. | n.b. |
| n.b. = nicht bestimmt | | | |

4. Adjuvans induzierte Polyarthritis
   Die Untersuchungen werden nach den Methoden von Pearson und Perper [Pearson, C.M. and F.D. Wood, Arth. Rheum. 2, 44 (1959); Perper, R.J. et al., Proc. Soc. exp. Biol. Med. 137, 506 1971)] durchgeführt. Als Versuchstiere dienen männliche Wistar-Lewis Ratten (Mollegaard, Dänemark) mit einem Körpergewicht zwischen 160 und 200 g. Polyarthritis wird durch Injektion von 0,1 ml Freund's Adjuvans (entspricht etwa 6 mg Mycobacterium butyricum Suspension, Difco Labs./Detroit, pro ml in Paraffinöl, Merck/Darmstadt) in die linke hintere Pfote induziert. Durch die Injektion kommt es zu immunpathologischen Prozessen, die zu chronischen Entzündungen innerhalb von 10 bis 14 Tagen führen. Es kommt insbesondere zu poly- und periarthritischen Symptomen in anderen Körperteilen und auch in der rechten hinteren Pfote.
   Die Prüfsubstanzen werden in CMC-Suspension oral verabreicht. Das Injektionsvolumen beträgt 1 mg/100 g Körpergewicht der Ratten. Die Verabreichung der erfindungsgemäßen Verbindungen erfolgt an 12 aufeinanderfolgenden Tagen, beginnend mit der Adjuvans-Injektion. 21 Tage nach Injektion wird das Pfotenvolumen beider Hinterpfoten gemessen. Als Kontrolle dienen CMC-Suspensionen.
   Als Beurteilungskriterium dient das Pfotenvolumen im Vergleich zur Kontrollgruppe. Die Hemmung der Pfotenvolumenzunahme wird in Prozentwerten angegeben. Die Tabelle 2 zeigt die Ergebnisse; in Klammern ist die Dosis der erfindungsgemäßen Verbindungen in mg/kg angegeben.

**Tabelle 2**

| Beispiel Nr. | Hemmung der Pfotenvolumenzunahme | |
|---|---|---|
| | linke Pfote (injiziert) | rechte Pfote (nicht injiziert) |
| 1 | 65 (25) | 87 (25) |
| 2 | 0 (25) | 31 (25) |
| 3 | 14 (25) | 0 (25) |
| 4 | 15 (25) | 0 (25) |
| 5 | 26 (25) | 34 (25) |
| 6 | 74 (25) | 87 (25) |

Abbauzeit in vivo
Die Prüfsubstanzen werden in CMC-Suspension oral verabreicht. Das Volumen beträgt 1 mg/100 g Körpergewicht der Ratten und 3 mg/100 g Körpergewicht der Mäuse. Als Vergleichssubstanz wird N-(4-Trifluormethylphenyl)-2-cyano-3-hydroxycrotonsäureamid verwendet. Die Bestimmung der Konzentration im Serum von der Vergleichssubstanz und den Prüfsubstanzen erfolgte nach der oralen Verabreichung der Substanzen.

Tabelle 3 zeigt die Ergebnisse.

**Tabelle 3**

| Prüfsubstanz Beispiel Nr. | | Konzentration (µg/ml) | | | | | |
|---|---|---|---|---|---|---|---|
| Zeit (Stunden) | | 1 | 3 | 6 | 24 | 48 | 72 |
| 4 | Maus | 67,7 | 59,3 | 45,4 | 0 | 0 | 0 |
| | Ratte | 2,4 | 1,4 | 0,2 | 0 | 0 | 0 |
| 6 | Maus | 64,5 | 85,0 | 76,7 | 0,44 | 0,1 | 0,1 |
| | Ratte | 11,6 | 22,6 | 25,4 | 0 | 0 | 0 |

| Vergleichssubstanz | | | | | | | |
|---|---|---|---|---|---|---|---|
| Maus | | 114,4 | 126,7 | 125,9 | 113,2 | 62,6 | 36,7 |
| Ratte | | 20,3 | 42,8 | 43,4 | 5,8 | 1,3 | 0,2 |

## Patentansprüche

1. Verwendung von mindestens einem N-Phenyl-2-cyano-3-hydroxycrotonsäureamid der Formel I 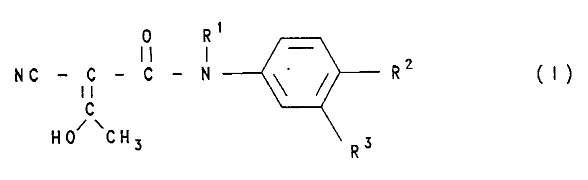 und/oder mindestens einem seiner physiologisch verträglichen Salze, wobei
R¹ ist:
a) Wasserstoffatom oder
b) (C₁-C₄)-Alkyl, geradkettig oder verzweigt,
R² ist ein Rest aus der Gruppe:
a) W(CH₂)ₙCX₃,
W ist:
1) Sauerstoffatom oder
2) Schwefelatom,
X ist Fluor, Chlor oder Jod,
n ist eine ganze Zahl von 1 bis 3,
b) WCX₃,
c) CX₃,
d) (CH₂)ₙCX₃,
e) Fluor, Chlor oder Jod,
f) CN oder
g) NO₂,
R³ ist ein Rest aus der Gruppe:
a) (C₁-C₄)-Alkyl, geradkettig oder verzweigt,
b) (C₃-C₆)-Cycloalkyl,
c) CN oder
d) 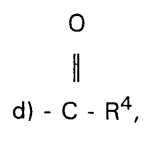
R⁴ ist:
1) Wasserstoffatom,
2) (C₁-C₄)-Alkyl, geradkettig oder verzweigt, oder
3) (C₃-C₆)-Cycloalkyl,
zur Herstellung von Arzneimitteln zur Behandlung von rheumatischen Erkrankungen, Autoimmunerkrankungen oder von Abstoßungsreaktionen des Organempfängers gegen das verpflanzte Organ.

2. Verwendung gemäß Anspruch 1, dadurch gekennzeichnet, daß man mindestens ein N-Phenyl-2-cyano-3-hydroxycrotonsäureamid der Formel I und/oder mindestens eines seiner physiologisch verträglichen Salze, wobei
R¹ ist Wasserstoff,
R² ist ein Rest aus der Gruppe:
a) CN,
b) Fluor, Chlor oder Jod,
c) CX₃,
X ist Fluor, Chlor oder Jod, oder
d) NO₂,
R³ ist ein Rest aus der Gruppe:
a) - CH₃,
b) - CN oder
c) 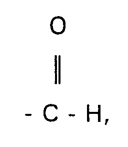
einsetzt.

3. Verwendung von
N-(3-Methyl-4-nitrophenyl)-2-cyano-3-hydroxycrotonsäureamid,
N-(3-Methyl-4-cyanophenyl)-2-cyano-3-hydroxycrotonsäureamid,
N-(3-Methyl-4-fluorphenyl)-2-cyano-3-hydroxycrotonsäureamid,
N-(3-Methyl-4-chlorphenyl)-2-cyano-3-hydroxycrotonsäureamid,
N-(3-Methyl-4-jodphenyl)-2-cyano-3-hydroxycrotonsäureamid oder
N-(3-Methyl-4-trifluormethylphenyl)-2-cyano-3-hydroxycrotonsäureamid
und/oder von deren physiologisch verträglichen Salzen zur Herstellung von Arzneimitteln zur Behandlung von rheumatischen Erkrankungen, Autoimmunerkrankungen oder von Abstoßungsreaktionen des Organempfängers gegen das verpflanzte Organ.

4. N-Phenyl-2-cyano-3-hydroxycrotonsäureamid der Formel I 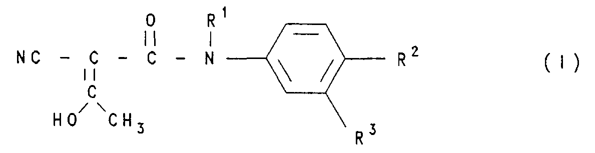 und/oder mindestens einem seiner physiologisch verträglichen Salze, wobei
R¹ ist:
a) Wasserstoffatom oder
b) (C₁-C₄)-Alkyl, geradkettig oder verzweigt,
R² ist ein Rest aus der Gruppe:
a) W(CH₂)ₙCX₃,
W ist:
1) Sauerstoffatom oder
2) Schwefelatom,
X ist Fluor, Chlor oder Jod,
n ist eine ganze Zahl von 1 bis 3,
b) WCX₃,
c) CX₃,
d) (CH₂)ₙCX₃,
e) Fluor, Chlor oder Jod,
f) CN oder
g) NO₂,
R³ ist (C₃-C₆)-Cycloalkyl oder 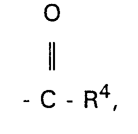 R⁴ ist:
1) Wasserstoffatom,
2) (C₁-C₄)-Alkyl, geradkettig oder verzweigt, oder
3) (C₃-C₆)-Cycloalkyl.

5. N-Phenyl-2-cyano-3-hydroxycrotonsäureamid der Formel I gemäß Anspruch 4 und/oder mindestens eines seiner physiologisch verträglichen Salze, wobei
R¹ ist Wasserstoff,
R² ist ein Rest aus der Gruppe:
a) CN,
b) Fluor, Chlor oder Jod,
c) CX₃,
X ist Fluor, Chlor oder Jod, oder
d) NO₂,
R³ ist
a) Cyclopropyl oder
b) 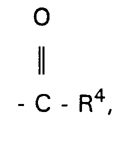
R⁴ ist Cyclopropyl.

6. Verfahren zur Herstellung von N-Phenyl-2-cyano-3-hydroxycrotonsäureamid der Formel I gemäß der Ansprüche 4 und 5 dadurch gekennzeichnet, daß man
a) eine Verbindung der Formel II, 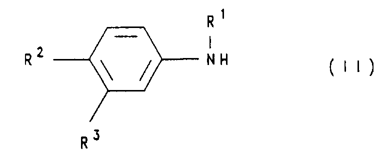 mit einer Verbindung der Formel III, 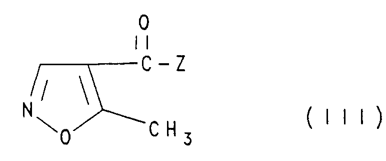 in der Z für ein Halogenatom, vorzugsweise Chlor oder Brom steht und R¹, R² und R³ die in Formel I gemäß der Ansprüche 4 und 5 angegebene Bedeutung haben, umsetzt und
b) die erhaltene Verbindung in Gegenwart eines basischen Mittels umsetzt.

7. Arzneimittel, gekennzeichnet durch einen wirksamen Gehalt von mindestens einem N-Phenyl-2-cyano-3-hydroxycrotonsäureamid der Formel I gemäß den Ansprüchen 4 und 5 und/oder mindestens einem seiner physiologisch verträglichen Salze.

8. Verfahren zur Herstellung eines Arzneimittels gemäß Anspruch 7, dadurch gekennzeichnet, daß man mindestens ein N-Phenyl-2-cyano-3-hydroxycrotonsäureamid der Formel I gemäß einem oder mehreren der Ansprüche 4 und 5 und/oder mindestens einem seiner physiologisch verträglichen Salze mit einem physiologisch annehmbaren Träger und weiteren geeigneten Wirk-, Zusatz- oder Hilfsstoffen in eine geeignete Darreichungsform bringt.

## Claims

1. The use of at least one N-phenyl-2-cyano-3-hydroxycrotonamide of the formula I 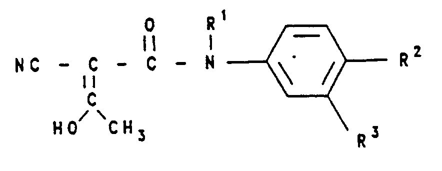 and/or of at least one of its physiologically tolerable salts, in which
R¹ is:
a) a hydrogen atom or
b) (C₁-C₄)-alkyl, which is straight-chain or branched,
R² is a radical of the group:
a) W(CH₂)ₙCX₃,
W is:
1) an oxygen atom or
2) a sulfur atom,
X is fluorine, chlorine or iodine,
n is an integer from 1 to 3,
b) WCX₃,
c) CX₃,
d) (CH₂)ₙCX₃,
e) fluorine, chlorine or iodine,
f) CN or
g) NO₂,
R³ is a radical of the group:
a) (C₁-C₄)-alkyl, which is straight-chain or branched,
b) (C₃-C₆)-cycloalkyl,
c) CN or
d) 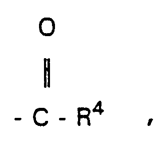
R⁴ is:
1) a hydrogen atom,
2) (C₁-C₄)-alkyl, which is straight-chain or branched, or
3) (C₃-C₆)-cycloalkyl,
for the production of pharmaceuticals for the treatment of rheumatic diseases, autoimmune diseases or of rejection reactions of the organ recipient to the transplanted organ.

2. The use as claimed in claim 1, wherein at least one N-phenyl-2-cyano-3-hydroxycrotonamide of the formula I and/or at least one of its physiologically tolerable salts is used, in which
R¹ is hydrogen,
R² is a radical of the group:
a) CN,
b) fluorine, chlorine or iodine,
c) CX₃,
X is fluorine, chlorine or iodine, or
d) NO₂,
R³ is a radical of the group:
a) -CH₃,
b) -CN or
c)

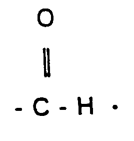

3. The use of
N-(3-methyl-4-nitrophenyl)-2-cyano-3-hydroxycrotonamide,
N-(3-methyl-4-cyanophenyl)-2-cyano-3-hydroxycrotonamide,
N-(3-methyl-4-fluorophenyl)-2-cyano-3-hydroxycrotonamide,
N-(3-methyl-4-chlorophenyl)-2-cyano-3-hydroxycrotonamide,
N-(3-methyl-4-iodophenyl)-2-cyano-3-hydroxycrotonamide or
N-(3-methyl-4-trifluoromethylphenyl)-2-cyano-3-hydroxycrotonamide
and/or of their physiologically tolerable salts for the production of pharmaceuticals for the treatment of rheumatic diseases, autoimmune diseases or of rejection reactions of the organ recipient to the transplanted organ.

4. An N-phenyl-2-cyano-3-hydroxycrotonamide of the formula I 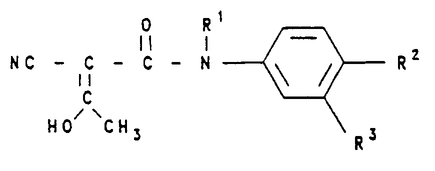 and/or at least one of its physiologically tolerable salts, in which
R¹ is:
a) a hydrogen atom or
b) (C₁-C₄)-alkyl, which is straight-chain or branched,
R² is a radical of the group:
a) W(CH₂)ₙCX₃,
W is:
1) an oxygen atom or
2) a sulfur atom,
X is fluorine, chlorine or iodine,
n is an integer from 1 to 3,
b) WCX₃,
c) CX₃,
d) (CH₂)ₙCX₃,
e) fluorine, chlorine or iodine,
f) CN or
g) NO₂,
R³ is (C₃-C₆)-cycloalkyl or 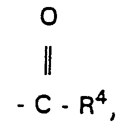 R⁴ is:
1) a hydrogen atom,
2) (C₁-C₄)-alkyl, which is straight-chain or branched, or
3) (C₃-C₆)-cycloalkyl.

5. An N-phenyl-2-cyano-3-hydroxycrotonamide of the formula I as claimed in claim 4 and/or at least one of its physiologically tolerable salts, in which
R¹ is hydrogen,
R² is a radical of the group:
a) CN,
b) fluorine, chlorine or iodine,
c) CX₃,
X is fluorine, chlorine or iodine, or
d) NO₂,
R³ is
a) cyclopropyl or
b) 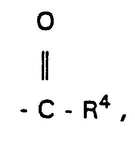
R⁴ is cyclopropyl.

6. A process for the preparation of N-phenyl-2-cyano-3-hydroxycrotonamide of the formula I as claimed in claims 4 and 5, which comprises
a) reacting a compound of the formula II 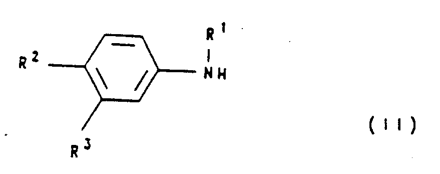 with a compound of the formula III 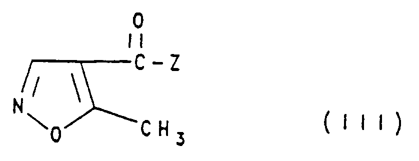 in which Z is a halogen atom, preferably chlorine or bromine, and R¹, R² and R³ have the meaning given in formula I as claimed in claims 4 and 5, and
b) reacting the compound obtained in the presence of a basic agent.

7. A pharmaceutical, which comprises an effective content of at least one N-phenyl-2-cyano-3-hydroxycrotonamide of the formula I as claimed in claims 4 and 5 and/or at least one of its physiologically tolerable salts.

8. A process for the production of a pharmaceutical as claimed in claim 7, which comprises bringing at least one N-phenyl-2-cyano-3-hydroxycrotonamide of the formula I as claimed in one or more of claims 4 and 5 and/or at least one of its physiologically tolerable salts into a suitable administration form with a physiologically acceptable excipient and other suitable active substances, additives or auxiliaries.

## Revendications

1. Utilisation d'au moins un N-phényl-2-cyano-3-hydroxycrotonamide de formule I 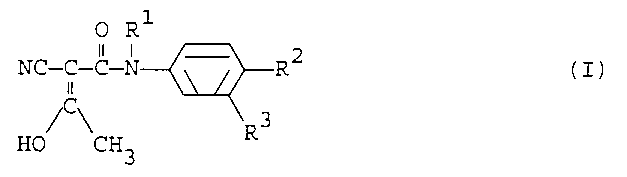 et/ou au moins un de ses sels physiologiquement acceptables, formule dans laquelle
R¹ est
a) un atome d'hydrogène ou
b) un groupe alkyle en C₁-C₄, à chaîne droite ou ramifiée,
R² est un radical choisi parmi:
a) W(CH₂)ₙCX₃,
W est:
1) un atome d'oxygène ou
2) un atome de soufre,
X est un atome de fluor, de chlore ou d'iode,
n est un nombre entier de 1 à 3,
b) WCX₃,
c) CX₃,
d) (CH₂)ₙCX₃,
e) un atome de fluor, de chlore ou d'iode,
f) CN ou
g) NO₂,
R³ est un radical choisi parmi:
a) un groupe alkyle en C₁-C₄, à chaîne droite ou ramifiée,
b) un groupe cycloalkyle en C₃-C₆,
c) CN ou
d) 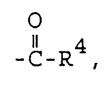 R⁴ est:
1) un atome d'hydrogène,
2) un groupe alkyle en C₁-C₄, à chaîne droite ou ramifiée, ou
3) un groupe cycloalkyle en C₃-C₆,
pour la fabrication de médicaments destinés au traitement de maladies rhumatismales, de maladies auto-immunes et de réactions de rejet par le receveur d'organe contre l'organe transplanté.

2. Utilisation selon la revendication 1, caractérisée en ce que l'on utilise au moins un N-phényl-2-cyano-3-hydroxycrotonamide de formule I et/ou au moins un de ses sels physiologiquement acceptables, dans lequel
R¹ est un atome d'hydrogène,
R² est un radical choisi parmi
a) CN,
b) un atome de fluor, de chlore ou d'iode,
c) CX₃,
X est un atome de fluor, de chlore ou d'iode, ou
d) NO₂,
R³ est un radical choisi parmi:
a) -CH₃,
b) -CN ou
c)

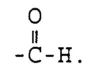

3. Utilisation du
N-(3-méthyl-4-nitrophényl)-2-cyano-3-hydroxycrotonamide,
N-(3-méthyl-4-cyanophényl)-2-cyano-3-hydroxycrotonamide,
N-(3-méthyl-4-fluorophényl)-2-cyano-3-hydroxycrotonamide,
N-(3-méthyl-4-chlorophényl)-2-cyano-3-hydroxycrotonamide,
N-(3-méthyl-4-iodophényl)-2-cyano-3-hydroxycrotonamide ou du
N-(3-méthyl-4-trifluorométhylphényl)-2-cyano-3-hydroxycrotonamide
et/ou de leurs sels physiologiquement acceptables, pour la fabrication de médicaments destinés au traitement de maladies rhumatismales, de maladies auto-immunes ou de réactions de rejet par le receveur d'organe contre l'organe transplanté.

4. N-phényl-2-cyano-3-hydroxycrotonamide de formule I 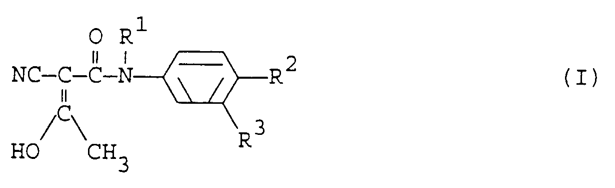 et/ou au moins un de ses sels physiologiquement acceptables, dans lequel
R¹ est:
a) un atome d'hydrogène ou
b) un groupe alkyle en C₁-C₄, à chaîne droite ou ramifiée,
R² est un radical choisi parmi:
a) W(CH₂)ₙCX₃,
W est:
1) un atome d'oxygène ou
2) un atome de soufre,
X est un atome de fluor, de chlore ou d'iode,
n est un nombre entier de 1 à 3,
b) WCX₃,
c) CX₃,
d) (CH₂)ₙCX₃,
e) un atome de fluor, de chlore ou d'iode,
f) CN ou
g) NO₂,
R³ est un groupe cycloalkyle en C₃-C₆ ou 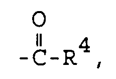 R⁴ est:
1) un atome d'hydrogène,
2) un groupe alkyle en C₁-C₄, à chaîne droite ou ramifiée, ou
3) un groupe cycloalkyle en C₃-C₆.

5. N-phényl-2-cyano-3-hydroxycrotonamide de formule I selon la revendication 4 et/ou au moins un de ses sels physiologiquement acceptables, dans lequel
R¹ est un atome d'hydrogène,
R² est un radical choisi parmi:
a) CN,
b) un atome de fluor, de chlore ou d'iode,
c) CX₃,
X est un atome de fluor, de chlore ou d'iode, ou
d) NO₂,
R³ est
a) le groupe cyclopropyle ou
b) 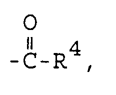 R⁴ est le groupe cyclopropyle.

6. Procédé pour la préparation d'un N-phényl-2-cyano-3-hydroxycrotonamide de formule I selon les revendications 4 et 5, caractérisé en ce que
a) on fait réagir un composé de formule II 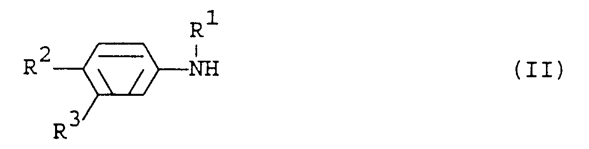 avec un composé de formule III 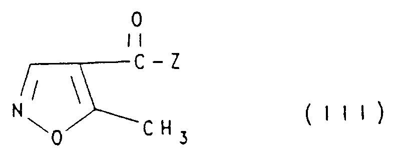 formules dans lesquelles Z représente un atome d'halogène, de préférence de chlore ou de brome, et R¹, R² et R³ ont les significations indiquées dans la formule I selon les revendications 4 et 5, et
b) on fait réagir en présence milieu basique le composé obtenu.

7. Médicament, caractérisé par une teneur efficace en au moins un N-phényl-2-cyano-3-hydroxycrotonamide de formule I selon les revendications 4 et 5, et/ou en au moins un de ses sels physiologiquement acceptables.

8. Procédé pour la fabrication d'un médicament selon la revendication 7, caractérisé en ce que l'on met sous une forme d'administration appropriée au moins un N-phényl-2-cyano-3-hydroxycrotonamide de formule I selon une ou plusieurs des revendications 4 et 5 et/ou au moins un de ses sels physiologiquement acceptables, avec un véhicule physiologiquement acceptable et d'autres substances actives, additifs ou adjuvants appropriés.
